# EUROPEAN PATENT APPLICATION

(11) **EP 4 454 641 A1**
(43) Date of publication of application: **30.10.2024**
(21) Application number: 22911687.6
(22) Date of filing: 06.12.2022
(51) Int. Cl.: A61K 9/24, A61K 9/20, A61K 31/4439, A61K 33/00, A61K 33/08, A61P 1/04

(54) **METHOD FOR PREPARING ORAL COMPOSITE TABLET CONTAINING PROTON PUMP INHIBITOR AND ANTACID AND ORAL COMPOSITE TABLET PREPARED THEREBY**

(30) Priority: 24.12.2021 KR 20210186751
(71) Applicant: Hanmi Pharm. Co., Ltd., Hwaseong-si, Gyeonggi-do 18536 (KR)
(72) Inventor: TAK, Jin Wook, Hwaseong-si, Gyeonggi-do 18536 (KR); KIM, Young Il, Hwaseong-si, Gyeonggi-do 18536 (KR); KWON, Taek Kwan, Hwaseong-si, Gyeonggi-do 18536 (KR); IM, Ho Taek, Hwaseong-si, Gyeonggi-do 18536 (KR); KIM, Yong Il, Hwaseong-si, Gyeonggi-do 18536 (KR)
(74) Representative: dompatent von Kreisler Selting Werner - Partnerschaft von Patent- und Rechtsanwälten mbB
(86) International application number: PCT/KR2022/019656
(87) International publication number: WO 2023/121054

(57) **Abstract**

The present invention relates to a method for preparing an oral composite tablet containing a proton pump inhibitor and an antacid and an oral composite tablet prepared thereby, and more specifically, to a method for preparing an oral composite tablet with excellent appearance stability and high productivity.

## Description

### [Technical Field]

This application claims the benefit of priority based on Korean Patent Application No. 10-2021-0186751 filed on December 24, 2021, and entire contents of which are incorporated as part of this specification.

The present invention relates to a method for preparing an oral composite tablet containing a proton pump inhibitor and an antacid and an oral composite tablet prepared thereby, and more specifically, to a method for preparing an oral composite tablet with excellent appearance stability and high productivity.

### [Background Art]

Digestive tract diseases such as peptic ulcer and gastroesophageal reflux disease occur when factors that attack the mucous membranes of the esophagus, stomach, and duodenum become more dominant than factors that protect them, and rapid gastric acid suppression is required to protect digestive tract diseases from gastric acid attack.

A proton pump inhibitor is a drug that inhibits the proton pump (H+/K+-ATPase) of parietal cells, suppressing the production of hydrochloric acid and weakening the strength of acidity in the digestive tract. It is effective in treating indigestion, gastric/esophageal reflux disease, pharyngeal/laryngeal reflux disease, or peptic ulcer disease. In particular, benzimidazole-based compounds or their salts are used as treatments for peptic ulcers with proton pump inhibitory action, examples of which include omeprazole, lansoprazole, rabeprazole, pantoprazole, and esomeprazole.

However, such proton pump inhibitors have the problem of being easily decomposed or deformed under acidic conditions. For example, esomeprazole is susceptible to acid decomposition and is also affected by moisture, heat, organic solvents, and light, and thus the formulation stability and dissolution rate are likely to decrease. Therefore, in order to secure the acid resistance of these proton pump inhibitors, methods of stabilizing them by coating them with enteric polymers or using them in combination with antacid are being considered. However, when a proton pump inhibitor is manufactured in a single tablet form by simply mixing it with an antacid, the drug is denatured by the acid as the proton pump inhibitor is directly exposed to an acidic environment. Therefore, due to insufficient pharmacological activity, bioavailability may decrease or related substances may rapidly increase, causing side effects such as toxicity in the body. Meanwhile, proton pump inhibitors coated with enteric polymers generally have weak buffering properties and are very vulnerable to shock, so they not only easily collapse during the manufacturing process, but also are easily exposed to acidic environments when the coating layer is lost, making it impossible to prevent decomposition and exert drug efficacy.

Accordingly, in order to improve the acid stability of the proton pump inhibitor and secure the dissolution rate of each component, a composite preparation with a double-layer tablet structure in which the proton pump inhibitor and antacid are separated into different layers is being considered. In this case, due to the nature of the double-layer tablet formulation, which prevents decomposition of the proton pump inhibitor by disintegrating the antacid and controlling the pH in the stomach, the properties of the excipients used in each upper and lower layer are different, the properties of the excipients used in each upper and lower layer are different, so it is important to secure the appearance stability of the double-layer tablet composite formulation. However, due to differences in the physical properties of the excipients used in each layer, problems such as tableting failure and friability defects of the two-layer composite preparation occur, resulting in poor productivity. Therefore, there is a need for the development of manufacturing technology for oral composite tablets in the form of double-layer tablets that have stable tablet properties and can be mass-produced.

### [Prior Art Document]

### [Patent Document]

Korean Laid-open Patent Publication No. 2008-0005575(2008.01.14), Stabilized composition

### [Disclosure]

### [Technical Problem]

It is an object of the present invention to provide a method for mass-production of an oral composite tablet with a double-layer structure that can stably secure the tableting properties of each layer containing different active ingredients without deteriorating the chemical stability thereof, in manufacturing an oral composite tablet containing a proton pump inhibitor and an antacid as active ingredients.

In addition, it is another object of the present invention to provide an oral composite tablet containing a proton pump inhibitor and an antacid with excellent appearance stability and productivity.

### [Technical Solution]

In order to achieve the above objects, the present invention provides a method for preparing an oral composite tablet, which comprises the following steps of:
(1) preparing a first granule by dry granulating a mixture containing a proton pump inhibitor or a pharmaceutically acceptable salt thereof and excipient;
(2) preparing a second granule by dry granulating a mixture containing an antacid and excipient thereof;
(3) filling the second granule into a container using a double-layer tablet press and then first compressing the lower layer by applying a pre-pressure of 1 kN or less; and
(4) filling the first granule on the lower layer and then second compressing the upper layer by applying a main pressure of 25 to 35 kN.

In addition, the present invention provides an oral composite tablet prepared according to the method for preparing an oral composite tablet of the present invention, which comprises: an upper layer containing a proton pump inhibitor or a pharmaceutically acceptable salt thereof as an active ingredient; and a lower layer containing an antacid as an active ingredient.

### [Advantageous Effects]

The method for preparing an oral composite tablet of the present invention is to manufacture a double-layer tablet comprising an upper layer containing a proton pump inhibitor and a lower layer containing an antacid, by applying tableting pressure appropriate to the physicochemical properties of the excipient contained in each of the upper and lower layers. Therefore, it is possible to secure appearance stability and manufacture double-layer tablets with excellent productivity without tableting failure or poor friability.

### [Description of Drawings]

Figure 1 is a photograph of the appearance of the oral composite tablet manufactured in Example 3 and Comparative Example 2 during tableting ((a): Example 3, (b): Comparative Example 2).
Figure 2 is a photograph of the appearance of the oral composite tablet manufactured in Example 6 and Comparative Example 6 during tableting ((a): Example 6, (b): Comparative Example 6).
Figure 3 is a photograph of the appearance of the oral composite tablet manufactured in Example 6 and Comparative Example 6 during tableting ((a): Example 6 upon completion of coating, (b): Comparative Example 6 at the beginning of coating, (c): Comparative Example 6 upon completion of coating).
Figure 4 is a photograph of the damage characteristics when measuring the hardness of the oral composite tablet manufactured in Example 6 and Comparative Example 6 ((a): Example 6, (b): Comparative Example 6).
Figure 5 is a graph showing the total related substance content (%) according to the stress test for the composite preparations manufactured in Examples 4 to 7.
Figure 6 is a graph showing esomeprazole dissolution rate (%) according to dissolution time for the oral composite preparations prepared in Examples 4 to 7.
Figure 7 is a graph showing the magnesium hydroxide dissolution rate (%) according to dissolution time for the oral composite preparations prepared in Examples 4 to 7.

### [Best Mode]

Hereinafter, the present invention will be described in more detail.

Terms or words used in this specification and claims should not be construed as limited to their ordinary or dictionary meanings but be construed as meaning and concept consistent with the technical idea of the present invention based on the principle that the inventor can appropriately define the concept of the term in order to explain his or her invention in the best way.

The terms used in the present invention are only used to describe specific Examples and are not intended to limit the present invention. Singular expressions include plural expressions unless the context clearly dictates otherwise. In the present invention, terms such as 'include' or 'have' are intended to designate the presence of features, numbers, steps, operations, components, parts, or combinations thereof described in the specification, and one or more other features, and should be understood that the terms do not exclude in advance the possibility of the existence or addition of elements, numbers, steps, operations, components, parts, or combinations thereof.

The present invention relates to a method for manufacturing an oral composite tablet containing a proton pump inhibitor and an antacid, and specifically includes the steps of:
(1) preparing a first granule by dry granulating a mixture containing a proton pump inhibitor or a pharmaceutically acceptable salt thereof and excipient;
(2) preparing a second granule by dry granulating a mixture containing an antacid and excipient thereof;
(3) filling the second granule into a container using a double-layer tablet press and then first compressing the lower layer by applying a pre-pressure of 1 kN or less; and
(4) filling the first granule on the lower layer and then second compressing the upper layer by applying a main pressure of 25 to 35 kN.

In particular, the oral composite tablet according to the present invention has the property of preventing decomposition of the proton pump inhibitor contained in the upper layer (referred to as the first layer in the present invention) by disintegrating the lower layer (referred to as the second layer in the present invention) containing the antacid and adjusting the pH state. Therefore, the physicochemical properties of the excipients used in the upper layer and lower layer are completely different. Accordingly, the first and second granules used in each layer have different degrees of compression against the same external pressure, that is, different compressibility, so the appearance stability of the final manufactured double-layer tablet can vary greatly. Therefore, if the tableting pressure is not set appropriately for the nature of the excipient contained in each granule, the oral composite tablet according to the present invention in the form of a double-layer tablet is prone to tableting problems such as capping, laminating, cracks, and friability defects. Specifically, capping is a phenomenon in which the top of the composite tablet peels off into a hat shape when the composite tablet is ejected from the punch, and laminating is a phenomenon in which capping becomes severe and the composite tablet peels off in layers on the side instead of the top. Therefore, in the present invention, the tableting pressure during tableting of each layer was set to a specific range, which is suitable for the physicochemical properties of the excipients included in each of the upper and lower layers, so that an oral composite tablet with a double-layer tablet structure having appropriate hardness can be manufactured without tableting problems or abrasion defects. The manufacturing method according to the present invention not only does not affect the pharmacological effect of the active ingredient, but also has an excellent effect in improving appearance stability, and can secure high productivity as the actual process is easy to introduce.

Hereinafter, the method for preparing an oral composite tablet will be described in detail in each step.

First, step (1) is a step of preparing the first granule by dry granulating a mixture containing a proton pump inhibitor or a pharmaceutically acceptable salt thereof and excipient.

The proton pump inhibitor (PPI) refers to any drug that possesses pharmacological activity as an inhibitor of the proton pump (H⁺/K⁺-ATPase), and includes salts, esters, amides, enantiomers, isomers, tautomers, prodrugs, derivatives, etc. of known proton pump inhibitory drugs. The final stage of gastric acid secretion in the body is the release of the proton pump (H⁺/K⁺-ATPase) into the gastric lumen and the import of K ions into the gastric lumen. Proton pump inhibitors strongly inhibit gastric acid secretion by inhibiting this pump. The proton pump inhibitor may include one or more selected from the group consisting of esomeprazole, omeprazole, lansoprazole, rabeprazole, and pantoprazole. In one embodiment, the proton pump inhibitor may be esomeprazole.

The esomeprazole((S)-5-methoxy-2-[(4-methoxy-3,5-dimethylpyridin-2-yl)methylsulfinyl]-3H-benzoimidazole) refers to the (S)-optical isomer, which is known to have excellent safety and efficacy among the two optical isomers of omeprazole. Lansoprazole (2-{[3-methyl-4-(2,2,2-trifluoroethoxy)pyridin-2-yl]methanesulfinyl}-1H-1,3-benzodiazole) is commercially available under the product name Lanstone.

The pharmaceutically acceptable salt of the proton pump inhibitor is any pharmaceutically acceptable salt that can be commonly used in the art, and may be, for example, metal salts such as magnesium (Mg) salt, strontium (Sr) salt, lithium salt, sodium salt, potassium salt and calcium salt, or ammonium salt, but are not limited to these. Additionally, the proton pump inhibitor or its pharmaceutically acceptable salt may be used in the form of an anhydride or hydrate, such as monohydrate, dihydrate, or trihydrate. For example, the proton pump inhibitor or a pharmaceutically acceptable salt thereof may be esomeprazole magnesium salt or lansoprazole. For example, the proton pump inhibitor may be esomeprazole magnesium trihydrate.

The excipient may include one or more selected from the group consisting of a diluent, a disintegrant, a binder, and a lubricant.

The diluent refers to a substance that serves to increase the volume of the preparation. For example, the diluent may be one or more selected from the group consisting of dicalcium phosphate anhydrous, D-mannitol, starch, low-substituted hydroxypropyl cellulose (L-HPC), microcrystalline cellulose, sucrose, sorbitol, xylitol, and glucose, but is not limited thereto.

The lubricant refers to a substance that facilitates extrusion and release of tablets by improving the fluidity of powder particles, increasing the filling ability into the die, which is the lower part of the tablet press, and reducing friction between the powder particles and between the powder particles and the punch-die, which is the upper part of the tablet press. For example, the lubricant may be one or more selected from the group consisting of calcium stearate, colloidal silicon dioxide(fumed silica, Aerosil), glyceryl monostearate, glyceryl palmitostearate, magnesium stearate, sodium lauryl sulfate, sodium stearyl fumarate, zinc stearate, stearic acid, hydrogenated vegetable oil, polyethylene glycol, sodium benzoate, talc, corn starch and carnauba wax, but is not limited thereto.

The disintegrant refers to a substance that absorbs moisture and promotes disintegration of the preparation, and can be used to improve the dissolution of the active ingredient. For example, the disintegrant may be one or more selected from the group consisting of crospovidone, cross-linked carboxymethylcellulose sodium (Cross-linked CMC Na, C.CMC Na, or croscarmellose sodium), corn starch, carboxymethylcellulose calcium, sodium starch glycolate, low-substituted hydroxypropyl cellulose (L-HPC), polyvinylpyrrolidone, pregelatinized starch, alginic acid and sodium alginate, but is not limited thereto.

The binder refers to a substance used to cause adhesion of powder particles in the preparation. For example, the binder may be one or more selected from the group consisting of sodium carboxymethylcellulose, ethylcellulose, hydroxyethylcellulose, hydroxypropyl cellulose, hydroxypropylmethylcellulose, methylcellulose, polyvinylpyrrolidone, gelatin, pregelatinized starch and povidone, but is not limited thereto.

In one embodiment, the mixture in step (1) contains as an excipient at least one selected from the group consisting of dicalcium phosphate anhydrous, mannitol, hydroxypropyl cellulose, sodium stearyl fumarate, microcrystalline cellulose, starch, crospovidone, cross-linked sodium carboxymethyl cellulose and magnesium stearate.

The dry granulation in step (1) above can be performed according to a conventional dry granulation method known in the art. In one embodiment, in step (1), the dry granulation is used to manufacture the first granule using a roller compactor with a mixture containing a binder, a lubricant, and a disintegrant as an excipient along with the proton pump inhibitor, which is the active ingredient.

Next, step (2) is a step of manufacturing the second granule by dry granulating a mixture containing an antacid and excipient.

The antacid (antacid agent) refers to a compound that can relieve the typical heartburn feeling (or pyrosis) caused by acid hypersecretion. In addition, the antacid refers to a drug that acts directly on both excess acid in the stomach and gastroesophageal reflux, that is, by buffering the pH of the gastric mucosa, or indirectly, for example, by suppressing acid secretion from the stomach. For example, the antacid includes a substance that reduces all of the symptoms reported above either indirectly by inhibition of acid secretion at the gastric level or directly by virtue of their neutralizing effect on gastric acidity. The antacid may be one or more selected from the group consisting of magnesium hydroxide, magnesium oxide, sodium bicarbonate and potassium carbonate, and in one embodiment, the antacid may include one or more selected from the group consisting of magnesium hydroxide and magnesium oxide.

The pharmaceutically acceptable salt of the antacid is any pharmaceutically acceptable salt that can be commonly used in the art. For example, metal salts such as magnesium (Mg) salt, strontium (Sr) salt, lithium salt, sodium salt, potassium salt and calcium salt, or ammonium salt may be used, but are not limited thereto. Additionally, the antacid or its pharmaceutically acceptable salt may be used in the form of an anhydride or hydrate, such as monohydrate, dihydrate, or trihydrate.

The excipient may include one or more selected from the group consisting of a diluent, a disintegrant, a fluidizing agent, and a lubricant.

The diluent may be one or more selected from the group consisting of calcium hydrogen phosphate, dicalcium phosphate anhydrous, calcium carbonate, D-mannitol, starch, low-substituted hydroxypropyl cellulose (L-HPC), microcrystalline cellulose, sucrose, sorbitol, xylitol and glucose, but is not limited thereto.

The lubricant may be one or more selected from the group consisting of calcium stearate, colloidal silicon dioxide(fumed silica, Aerosil), glyceryl monostearate, glyceryl palmitostearate, magnesium stearate, sodium lauryl sulfate, sodium stearyl fumarate, zinc stearate, stearic acid, hydrogenated vegetable oil, polyethylene glycol, sodium benzoate, talc, corn starch and carnauba wax, but is not limited thereto.

The disintegrant may be one or more selected from the group consisting of crospovidone, cross-linked carboxymethylcellulose sodium (Cross-linked CMC Na, C.CMC Na, or croscarmellose sodium), corn starch, carboxymethylcellulose calcium, sodium starch glycolate, low-substituted hydroxypropyl cellulose (L-HPC), polyvinylpyrrolidone, pregelatinized starch, alginic acid and sodium alginate, but is not limited thereto.

The fluidizing agent refers to an ingredient that improves the flow characteristics of an active ingredient, excipient, or the mixture thereof included in the mixture of step (2) above, and may be included to maximize the effect without affecting other ingredients. For example, the fluidizing agent may be one or more selected from the group consisting of silicon dioxide, colloidal silicon dioxide, talc, and mixtures thereof, but is not limited thereto.

In one embodiment, the mixture in step (2) may include one or more excipients selected from the group consisting of dicalcium phosphate anhydrous, mannitol, hydroxypropyl cellulose, sodium stearyl fumarate, colloidal silicon dioxide, microcrystalline cellulose, pregelatinized starch, crospovidone, cross-linked sodium carboxymethyl cellulose and magnesium stearate.

The dry granulation in step (2) is the same as described in step (1). In one embodiment, in step (2), the dry granulation can produce the second granule using a roller compactor with a mixture containing antacid, the active ingredient, as well as a lubricant and a disintegrant as an excipient.

Next, step (3) is the step of filling the second granule into a container using a double-layer tablet press and then applying a pre-pressure of 1 kN or less to first tablet the lower layer (second layer).

In the oral composite tablet in the form of a double-layer tablet according to the present invention, the lower layer needs to have sufficient hardness and rapid disintegration. Therefore, in the present invention, after filling the second granule in a container, a pre-pressure of 1 kN or less is applied and compressed into tablets, thereby forming a lower layer with appropriate hardness and rapid disintegration characteristics.

In one embodiment, the pre-pressure of step (3) may be 0.1 to 1 kN. If the pre-pressure in step (3) above is less than 0.1 kN, hardness is insufficient and air in the filled second granule cannot be sufficiently removed, which may cause capping. On the contrary, if the pre-pressure in step (3) above exceeds 1 kN, the interlayer bonding force of the upper and lower layers may decrease, causing cracks or layer separation.

In step (3) above, the tableting process may be performed based on a typical double-layer tablet oral composite tablet manufacturing process performed in the relevant technical field.

Next, step (4) is a step of filling the first granule on the lower layer and then performing secondary compression of the upper layer by applying main pressure of 25 to 35 kN.

As described above, in the case of the oral composite tablet of the present invention having a double-layer tablet structure, appearance stability problems such as tableting failure and friability defects occur due to differences in tableting properties resulting from different physicochemical properties of the excipients included in each layer.

Therefore, in the present invention, as described above, in addition to applying a certain range of pre-pressure when forming the lower layer, the first granule is filled on the lower layer and then compressed by applying main pressure in the range of 25 to 35 kN to improve appearance stability and productivity by improving tableting difficulties and friability defects of oral composite tablets in the form of double-layer tablets.

In one embodiment, the main pressure in step (4) may be 25 to 35 kN or 28 to 35 kN. If the main pressure in step (4) is less than 25 kN, there is a problem of severe edge wear due to the low hardness of the composite tablet. On the contrary, if the main pressure in step (4) above exceeds 35 kN, the interlayer bonding force may decrease, causing layer separation, and tableting failure may occur.

In step (4) above, the tableting process can be performed based on the oral composite tablet manufacturing process having a typical double-layer tablet form performed in the relevant technical field.

The method for preparing an oral composite tablet according to the present invention may further include the steps of coating, selecting, and packaging the oral composite tablet obtained through steps (1) to (4) above.

As described above, in the method for preparing an oral composite tablet of the present invention, the pre-pressure of the second granule when compressing the lower layer is set to 1 kN or less, and the main pressure of the first granule is set to 25 to 35 kN when compressing the upper layer. By doing so, tableting difficulties do not occur and sufficient friability can be secured. Accordingly, the oral composite tablet manufactured according to the manufacturing method of the present invention can be formed by tableting in the form of a double-layer tablet with the first granule containing the proton pump inhibitor as the upper layer (first layer) and the second granule containing the antacid as the lower layer (second layer), and as a double-layer tablet, both tablet stability and dissolution characteristics of each active ingredient can be secured. Specifically, the oral composite tablet manufactured according to the manufacturing method of the present invention has the advantage of satisfying tablet stability because the active ingredients contained in the upper and lower layers exhibit the desired dissolution rate and have stable hardness as a double-layer tablet. In addition, since these oral composite tablets in the form of double-layer tablets can be mass-produced, the productivity of double-layer tablets can be improved while satisfying both manufacturing reliability and product reliability.

In addition, the present invention relates to an oral composite tablet containing a proton pump inhibitor and an antacid prepared from the above-described manufacturing method, and specifically, comprises:
an upper layer (first layer) containing a proton pump inhibitor or a pharmaceutically acceptable salt thereof as an active ingredient; and
a lower layer (second layer) containing an antacid as an active ingredient.

The oral composite tablet is manufactured by the above-described manufacturing method, and the description of the method for preparing an oral composite tablet according to the present invention can be applied as it is to the details of the upper layer and lower layer included in the oral composite tablet.

Additionally, any pharmaceutical additives commonly used in the art may be included in an appropriate amount. For example, one or more additives selected from the group consisting of surfactants, antioxidants, preservatives, stabilizers, flavoring agents, colorants, solubilizers, pH adjusters, coating agents, and any combinations thereof may be additionally included, but are not limited thereto.

The coating agent may be one or more selected from the group consisting of polyvinyl alcohol (PVA), hydroxypropylmethylcellulose (HPMC), and hydroxypropyl cellulose (HPC), but is not limited thereto.

The oral composite tablet may be a multi-layer tablet such as a double-layer tablet or a triple-layer tablet. In one embodiment, the oral composite tablet may be a double-layer tablet, and in this case, it may be composed of an upper layer (first layer) containing a proton pump inhibitor or a pharmaceutically acceptable salt thereof as the active ingredient and a disintegrant, and a lower layer (second layer) containing an antacid as the active ingredient and a disintegrant.

The oral composite tablet of the present invention is manufactured as a double-layer tablet according to the above-described manufacturing method, so that the active ingredients contained in the upper and lower layers exhibit the desired dissolution rate and have stable hardness as a double-layer tablet. In one embodiment, the oral composite tablet may have a hardness of 12.8 to 15.5 kp.

The oral composite tablet of the present invention according to one embodiment may be a multi-layer tablet, for example, a double-layer tablet, and the proton pump inhibitor and antacid present in different layers may exhibit temporal release for each layer. For example, in the oral composite tablet, the antacid present in the lower layer is released first to increase the pH in the stomach, and then the proton pump inhibitor present in the upper layer is released to neutralize the acidic environment in the stomach and prevent the decomposition of the proton pump inhibitor by gastric acid.

The oral composite tablet contains a proton pump inhibitor and an antacid as active ingredients, wherein, unlike commercial enteric preparations, the antacid quickly neutralizes gastric acid without enteric coating to prevent decomposition of the proton pump inhibitor, and the proton pump inhibitor is quickly dissolved and absorbed in the body, without delaying the onset of drug effect.

In addition, the oral composite tablet prevents decomposition of the proton pump inhibitor in the stomach due to the time-dependent release of the proton pump inhibitor and antacid and improves stability, thereby providing a pharmaceutical composite preparation that exhibits an optimal drug release pattern. In one embodiment, the total related substance content of the proton pump inhibitor or pharmaceutically acceptable salt thereof in the composite preparation is based on 2.0% by weight or less of the total weight of the proton pump inhibitor or pharmaceutically acceptable salt thereof. The total related substance content may be measured after 1 week, 2 weeks, and 4 weeks while stored in a chamber under stress stability conditions at 60°C.

In one embodiment, the oral composite tablet may have a proton pump inhibitor dissolution rate of 10% or more and 50% or less for 10 minutes and an antacid dissolution rate of 70% or more at the same time, when tested in a mixture of 75 ml of 0.1N HCl and 225 ml of purified water under conditions of a rotation speed of 75 rpm and 37±0.5 °C according to the paddle method, the second method of the United States Pharmacopoeia (USP) dissolution test.

Hereinafter, preferred examples are presented to aid understanding of the present invention. However, the following examples are merely illustrative of the present invention, and it is clear to those skilled in the art that various changes and modifications are possible within the scope and spirit of the present invention. It is also natural that such variations and modifications fall within the scope of the attached patent claims.

### Example and Comparative Example: Preparation of oral composite tablet

An oral composite tablet containing a proton pump inhibitor in the upper layer (first layer) and an antacid in the lower layer (second layer) was manufactured according to the composition and content shown in Tables 1 and 2 below.

Specifically, esomeprazole magnesium trihydrate was mixed with microcrystalline cellulose, crospovidone, hydroxypropyl cellulose, and sodium stearyl fumarate for 30 minutes. The prepared mixture was made into flakes using a roller compactor under the conditions of a roller speed of 25 rpm, hydraulic pressure of 2 MPa, and roller gap of 2.5 mm, and then sieved through a sieve with an opening size of 1.0 mm to prepare dry granules. The first granule for forming the upper layer (first layer) was prepared by mixing sodium stearyl fumarate as a lubricant with the prepared dry granule for 5 minutes.

Magnesium hydroxide was mixed with microcrystalline cellulose and crospovidone for 24 minutes. The prepared mixture was made into flakes using a roller compactor under the conditions of a roller speed of 25 rpm, hydraulic pressure of 2 MPa, and roller gap of 2.5 mm, and then sieved through a sieve with an opening size of 2.0 mm to prepare dry granules. As a lubricant, colloidal silicon oxide was distributed into the prepared dry granules, sieved through a sieve with an opening size of 0.8 mm, mixed for 24 minutes, and mixed with sodium stearyl fumarate as a lubricant for 6 minutes to prepare second granules to form a lower layer (second layer).

The lower layer was first tableted by applying the pre-pressure shown in Tables 1 and 2 below to the prepared second granules using a double-layer tablet press and then the upper layer was second tableted by applying the main pressure shown in Tables 1 and 2 below to the prepared first granules using a double-layer tablet press, and skin coating was performed.

Specifically, tableting was performed using a double-layer tablet press with a tableting speed of 10 rpm, lower layer supply speed of 100 rpm, and upper layer supply speed of 100 rpm, and coating was performed at a rotation speed of 2 rpm, a supply air temperature of 53°C, and an exhaust temperature of 40°C.

**[Table 1]**

| Ingredient (Unit: mg) | | Example 1 | Example 2 | Example 3 | Example 4 | Example 5 | Example 6 | Example 7 |
|---|---|---|---|---|---|---|---|---|
| Upper layer (First layer) | Esomeprazole magnesium trihydrate | 44.5 | 44.5 | 44.5 | 44.5 | 44.5 | 44.5 | 44.5 |
| | Microcrystalline cellulose | 227.5 | 227.5 | 227.5 | 227.5 | 227.5 | 227.5 | 227.5 |
| | Hydroxypropyl cellulose | 32.0 | 32.0 | 32.0 | 32.0 | 32.0 | 32.0 | 32.0 |
| | Crospovidone | 8.0 | 8.0 | 8.0 | 8.0 | 8.0 | 8.0 | 8.0 |
| | Sodium stearyl fumarate | 18.0 | 18.0 | 18.0 | 18.0 | 18.0 | 18.0 | 18.0 |
| | Upper layer total content | 330.0 | 330.0 | 330.0 | 330.0 | 330.0 | 330.0 | 330.0 |
| Lower layer (Second layer) | Magnesium hydroxide | 350.0 | 350.0 | 350.0 | 350.0 | 350.0 | 350.0 | 350.0 |
| | Microcrystalline cellulose | 135.0 | 135.0 | 135.0 | 135.0 | 135.0 | 135.0 | 135.0 |
| | Crospovidone | 15.0 | 15.0 | 15.0 | 15.0 | 15.0 | 15.0 | 15.0 |
| | Colloidal silicon oxide | 15.0 | 15.0 | 15.0 | 15.0 | 15.0 | 15.0 | 15.0 |
| | Sodium stearyl fumarate | 5.0 | 5.0 | 5.0 | 5.0 | 5.0 | 5.0 | 5.0 |
| | Lower layer total content | 520 | 520 | 520 | 520.0 | 520.0 | 520.0 | 520.0 |
| Naked tablet total content | | 850.0 | 850.0 | 850.0 | 850.0 | 850.0 | 850.0 | 850.0 |
| Tablet pressure (Unit: kN) | lower layer pre-pressure | 0.1 | 0.5 | 1 | 1 | 1 | 1 | 1 |
| | upper layer main pressure | 30 | 30 | 30 | 25 | 28 | 32 | 35 |

**[Table 2]**

| Ingredient (Unit: mg) | | Compara tive Example 1 | Compara tive Example 2 | Compara tive Example 3 | Compara tive Example 4 | Compara tive Example 5 | Compara tive Example 6 |
|---|---|---|---|---|---|---|---|
| Upper layer (First layer) | Esomeprazole magnesium trihydrate | 44.5 | 44.5 | 44.5 | 44.5 | 44.5 | 44.5 |
| | Microcrystalline cellulose | 227.5 | 227.5 | 227.5 | 227.5 | 227.5 | 227.5 |
| | Hydroxypropyl cellulose | 32.0 | 32.0 | 32.0 | 32.0 | 32.0 | 32.0 |
| | Crospovidone | 8.0 | 8.0 | 8.0 | 8.0 | 8.0 | 8.0 |
| | Sodium stearyl fumarate | 18.0 | 18.0 | 18.0 | 18.0 | 18.0 | 18.0 |
| | Upper layer total content | 330.0 | 330.0 | 330.0 | 330.0 | 330.0 | 330.0 |
| Lower layer (Second layer) | Magnesium hydroxide | 350.0 | 350.0 | 350.0 | 350.0 | 350.0 | 350.0 |
| | Microcrystalline cellulose | 135.0 | 135.0 | 135.0 | 135.0 | 135.0 | 135.0 |
| | Crospovidone | 15.0 | 15.0 | 15.0 | 15.0 | 15.0 | 15.0 |
| | Colloidal silicon oxide | 15.0 | 15.0 | 15.0 | 15.0 | 15.0 | 15.0 |
| | Sodium stearyl fumarate | 5.0 | 5.0 | 5.0 | 5.0 | 5.0 | 5.0 |
| | Lower layer total content | 520.0 | 520.0 | 520.0 | 520.0 | 520.0 | 520.0 |
| Naked tablet total content | | 850.0 | 850.0 | 850.0 | 850.0 | 850.0 | 850.0 |
| Tablet pressure (Unit: kN) | lower layer pre-pressure | 3 | 5 | 1 | 1 | 1 | 1 |
| | upper layer main pressure | 30 | 30 | 19 | 22 | 38 | 40 |

### Test Example 1. Evaluation of characteristics of oral composite tablet according to lower layer pre-pressure

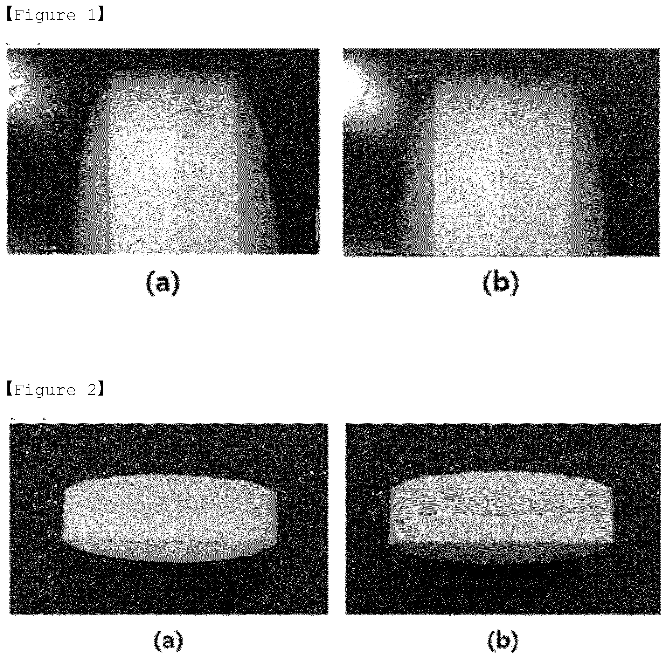

Tests were conducted to evaluate the hardness, appearance defect (capping, laminating, interlayer crack) ratio during tableting and coating and friability of oral composite tablets manufactured in Examples 1 to 3 and Comparative Examples 1 and 2 above, and the results are shown in Table 3 and Figure 1 below.

Hardness was measured using a tablet hardness tester (ERWEKA GmBH Tablet hardness tester), and appearance defect measurement was performed by randomly selecting 20 tablets during the tableting and coating process. In the case of friability measurement, based on the Korean Pharmacopoeia, it was measured at 25 rpm for 4 minutes and a total of 100 rotations were set to 1 time, and was calculated as the difference in mass before and after friability measurement. In general, in the above friability measurement, 1-time friability is used as an evaluation item to check tableting failure in the tableting process, and 4-time friability is used as an evaluation item to ensure tablet appearance stability within the coating machine. The evaluation standard for 1-time friability is within 0.5%, and the evaluation standard for 4-time friability is within 2.0%.

**[Table 3]**

| Evaluation items | | Example 1 | Example 2 | Example 3 | Comparative Example 1 | Comparative Example 2 |
|---|---|---|---|---|---|---|
| Lower layer pre-pressure (Unit: kN) | | 0.1 | 0.5 | 1 | 3 | 5 |
| Hardness (Unit: kp) | | 12.8 | 13.5 | 14.2 | 15.8 | 16.7 |
| Appearance defect ratio | Tableting | 0 / 20 | 0 / 20 | 0 / 20 | 2 / 20 | 5 / 20 |
| | Coating | 0 / 20 | 0 / 20 | 0 / 20 | 4 / 20 | 8 / 20 |
| Friability (Unit: %) | 1-time | 0.38 | 0.32 | 0.21 | 0.17 | 0.15 |
| | 4-time | 1.86 | 1.57 | 1.23 | 1.09 | 1.01 |

From the results in Table 3 above, good results within the standard were obtained when evaluating friability in both Examples 1 to 3 and Comparative Examples 1 and 2, and as the pre-pressure for the lower layer increases, relatively favorable results are obtained in terms of friability.

However, in Comparative Examples 1 and 2, where pre-pressure exceeding 1 kN was applied to the lower layer, the appearance defect ratio increased during tableting and coating. This appears to be due to differences in tableting properties of each layer due to different physicochemical properties of the excipients used in the upper and lower layers. In other words, due to this difference in compressibility, the tablets of Comparative Examples 1 and 2, in which appropriate pre-pressure was not applied to the lower layer, appear to have had interlayer cracks due to a decrease in interlayer bonding force (see Figure 1) .

### Test Example 2. Evaluation of characteristics of oral composite tablet according to upper layer main pressure

The oral composite tablets prepared in Examples 4 to 7 and Comparative Examples 3 and 6 were evaluated for hardness, appearance defect (capping, laminating, interlayer crack) ratio during tableting and coating, friability, and tablet drop test, and the results are shown in Table 4 and Figures 2 to 4 below.

The evaluation test for hardness, appearance defect, and friability was conducted in the same manner as Test Example 1 above. In the tablet drop test, the number of broken tablets was measured when each 30T HDPE bottle was dropped horizontally and vertically 10 times from a height of 1.5 m.

**[Table 4]**

| Evaluation items | | Example 4 | Example 5 | Example 6 | Example 7 | Compara tive Example 3 | Compara tive Example 4 | Compara tive Example 5 | Compara tive Example 6 |
|---|---|---|---|---|---|---|---|---|---|
| Upper layer main pressure (Unit: kN) | | 25 | 28 | 32 | 35 | 19 | 22 | 38 | 40 |
| Hardness (Unit: kp) | | 13.8 | 14.1 | 14.5 | 15.2 | 11.2 | 12.7 | 15.8 | 16.4 |
| Appearance defect rate | Tableting | 0 / 20 | 0 / 20 | 0 / 20 | 0 / 20 | 0 / 20 | 0 / 20 | 2 / 20 | 3 / 20 |
| | Coating | 0 / 20 | 0 / 20 | 0 / 20 | 0 / 20 | 0 / 20 | 0 / 20 | 4 / 20 | 4 / 20 |
| Friability (Unit: %) | 1 time | 0.39 | 0.27 | 0.20 | 0.17 | 0.67 | 0.52 | 0.15 | 0.12 |
| | 4 times | 1.79 | 1.44 | 1.21 | 1.17 | 2.19 | 2.01 | 1.13 | 1.02 |
| Damage rate when dropped | Width | 0 / 30 | 0 / 30 | 0 / 30 | 0 / 30 | 5 / 30 | 3 / 30 | 4 / 30 | 4 / 30 |
| | length | 0 / 30 | 0 / 30 | 0 / 30 | 0 / 30 | 3 / 30 | 2 / 30 | 3 / 30 | 6 / 30 |

Through Table 4 above, in the case of Examples 4 to 7 where the main pressure was applied in the range of 25 to 35 kN when tableting the upper layer, not only did no appearance defects occur during tableting and coating and no damage occurred when dropping, but also showed good friability evaluation results.

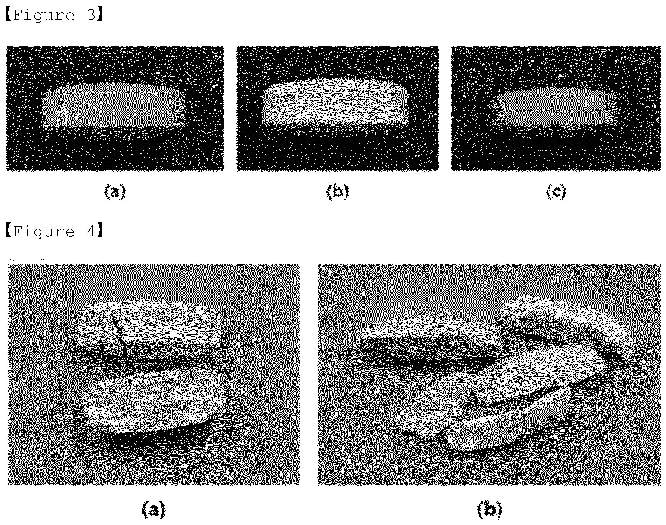

Meanwhile, in Comparative Examples 3 and 4 where the main pressure below 25 kN was applied during upper layer tableting, no appearance defect ratio occurred during tableting and coating, but it was confirmed that the final oral composite tablet did not have appropriate hardness, resulting in serious edge wear and poor friability. In addition, in Comparative Examples 5 and 6, where the main pressure over 35 kN was applied when compressing the upper layer, it was found that the bonding force between the upper layer and the lower layer was reduced, causing layer separation, which resulted in interlayer cracks. In addition, it was confirmed that the air in the finally manufactured oral composite tablet was not sufficiently released, resulting in capping or crack formation due to air being released to the boundary of the layer with weak bonding force during the preheating or coating process (see Figures 2 and 3). Even in the tablet drop test conducted in the packaging state, the oral composite tablets of Comparative Examples 3 to 6, which were outside the upper layer main pressure range set in the present invention, had a higher damage rate than Examples 4 to 7 due to low hardness or tableting failure. In addition, as shown in Figure 4, when measuring tablet hardness, it was confirmed that when an interlayer crack was present in the finally manufactured oral composite tablet as in Comparative Example 6, the pattern of damage was separated along the interlayer crack despite having a higher hardness than Example 6.

### Test Example 3. Evaluation of related substances of oral composite tablet

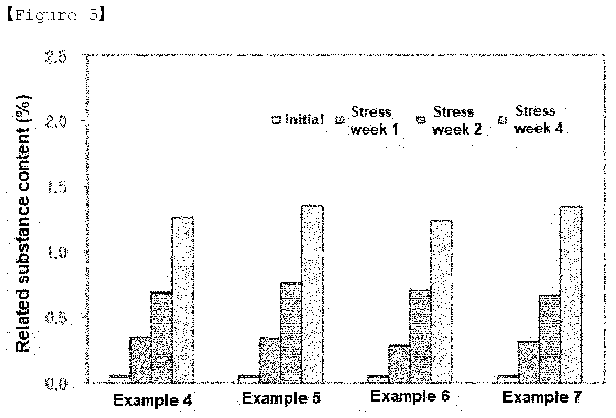

In order to confirm the stability of the oral composite tablets prepared in Examples 4 to 7 over time, the total content of related substances was measured for the oral composite tablets in Examples 4 to 7. Specifically, the oral composite tablets prepared in Examples 4 to 7 were each packaged in HDPE bottles and stored in a chamber under severe stability conditions at 60°C. At week 0 (initial), week 1, week 2, and week 4, the total content of related substances generated from each oral composite tablet was measured according to the analysis conditions below, and the results are shown in Figure 5.

### <Analysis condition>

Column: Hypersil BDS C18 100 mm x 4.6 mm, 3 µm (Column temperature: 25°C)
Mobile phase A: Acetonitrile: Phosphate buffer solution (pH 7.6): Water = 100: 100: 800
Mobile phase B: Acetonitrile: Phosphate buffer solution (pH 7.6): Water = 800: 10: 190

(The phosphate buffer solution (pH 7.6) in the Mobile phase A and Mobile phase B was made by mixing 5.2 ml of 1.0 mol/L sodium dihydrogen phosphate and 63.0 ml of 0.5 mol/L disodium hydrogen phosphate and adding water to make 1 L.)
Detector: Ultraviolet absorption spectrophotometer (measurement wavelength 302 nm)
Flow rate: 1.0 mℓ/minute
Injection volume: 20 µℓ
Sample temperature: 4 °C

As shown in Figure 5, the oral composite tablets manufactured in Examples 4 to 7 were found to have excellent stability, as the total content of related substance generated from each oral composite tablet satisfied the standard of 2.0% or less for up to Week 4 of stress.

### Test Example 4. Evaluation of esomeprazole dissolution characteristics

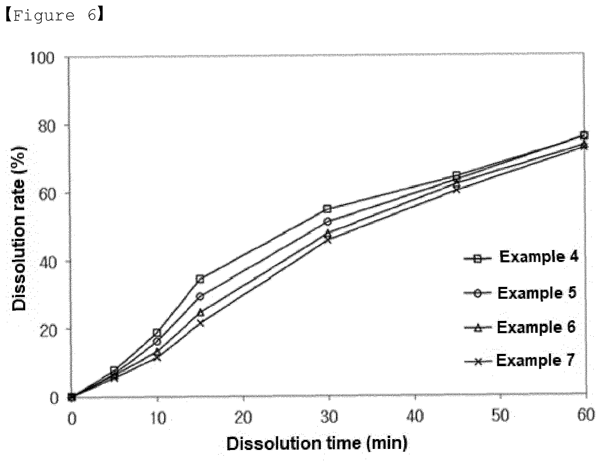

Under the following dissolution and analysis conditions, the esomeprazole dissolution rate of the oral composite tablets prepared in Examples 4 to 7 was evaluated, and the results are shown in Figure 6.

### <Dissolution condition>

Dissolution medium: A mixture of 75 ml of 0.1 N HCl and 225 ml of purified water
Test device: paddle method, the second method of USP dissolution test, 75 rpm
Temperature: 37 ± 0.5°C
Dissolution time: 5, 10, 15, 30, 45, 60 minutes
Test method: Two tablets (80 mg of esomeprazole) were added to the dissolution medium, and the dissolution medium collected over time was passed through a 0.45 µm membrane filter, and then immediately mixed with 0.25 M NaOH in a 5: 1 ratio to serve as the sample solution.

### <Analysis condition>

Column: Inertsil ODS-3V 150 mm x 4.6 mm, 5 µm
Column temperature: 25°C
Mobile phase: Acetonitrile: Phosphate buffer solution (pH 7.3): Water = 350: 500: 150
(Phosphate buffer solution (pH 7.3) was made by mixing 10.5 ml of 1 mol/L sodium dihydrogen phosphate and 60.0 ml of 0.5 mol/L disodium hydrogen phosphate and adding water to make 1 L.)
Detector: Ultraviolet absorption spectrophotometer
Measurement wavelength: 302 nm
Flow rate: 1.0 mℓ/minute
Injection volume: 20 µℓ
Sample temperature: 25°C

Under the above conditions, the solution mixed with 0.1 N HCl and purified water, which is the dissolution medium, reenacts the gastric juice (0.1 N HCl) present in the stomach and the water (purified water) taken together when taking the medicine. This is to check the degree of decomposition of esomeprazole, a proton pump inhibitor that can be decomposed at low pH and the antacid power of antacid when taken by humans.

From Figure 6, it was confirmed that the oral composite tablets according to Examples 4 to 7 showed an esomeprazole dissolution rate of about 10% or more for 10 minutes and an esomeprazole dissolution rate of about 70% or more for 60 minutes. Through this, it was found that the decomposition of esomeprazole by gastric acid was prevented, its stability was improved, and drug release was not reduced.

### Test Example 5. Evaluation of magnesium hydroxide dissolution characteristics

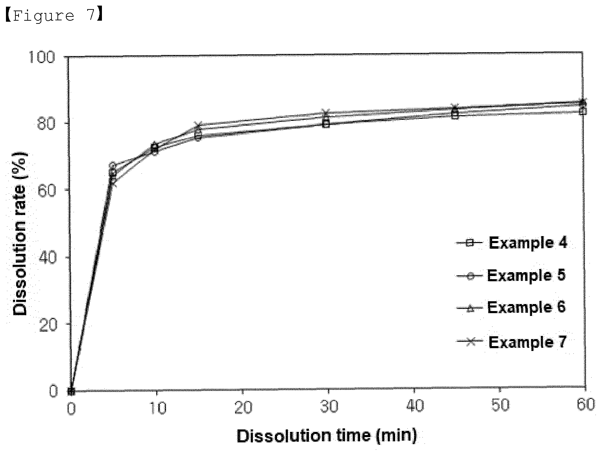

Under the following dissolution and analysis conditions, the magnesium hydroxide dissolution rate of the oral composite tablets prepared in Examples 4 to 7 was evaluated, and the results are shown in Figure 7.

### <Dissolution condition>

Dissolution medium: A mixture of 75 ml of 0.1 N HCl and 225 ml of purified water
Test device: paddle method, the second method of USP dissolution test, 75 rpm
Temperature: 37 ± 0.5°C
Dissolution time: 5, 10, 15, 30, 45, 60 minutes
Test method: 2 tablets (80 mg of esomeprazole) were tested by adding them to the dissolution medium.

### <Analysis condition>

Detector: Atomic Absorption Spectrophotometer
Lamp: Magnesium hollow cathode lamp
Wavelength: 285.2 nm
Gas used: Air-Acetylene

As shown in Figure 7, it was confirmed that magnesium hydroxide contained in the lower layer of the oral composite tablets manufactured in Examples 4 to 7 showed a dissolution rate of about 70% or more at 10 minutes.

In addition, when considering the results in Figures 6 and 7 together, it was found that the oral composite tablets according to Examples 4 to 7 effectively prevented acid decomposition of esomeprazole and showed a stable esomeprazole dissolution rate.

## Claims

1. A method for preparing an oral composite tablet, which comprises the following steps of:
(1) preparing a first granule by dry granulating a mixture containing a proton pump inhibitor or a pharmaceutically acceptable salt thereof and excipient;
(2) preparing a second granule by dry granulating a mixture containing an antacid or a pharmaceutically acceptable salt thereof and excipient;
(3) filling the second granule into a container using a double-layer tablet press and then first compressing the lower layer by applying a pre-pressure of 1 kN or less; and
(4) filling the first granule on the lower layer and then second compressing the upper layer by applying a main pressure of 25 to 35 kN.

2. The method for preparing an oral composite tablet according to claim 1, wherein the proton pump inhibitor includes one or more selected from the group consisting of esomeprazole, omeprazole, lansoprazole, rabeprazole and pantoprazole.

3. The method for preparing an oral composite tablet according to claim 1, wherein the antacid includes one or more selected from the group consisting of magnesium hydroxide, magnesium oxide, sodium bicarbonate and potassium carbonate.

4. The method for preparing an oral composite tablet according to claim 1, wherein the excipient of the step (1) includes one or more selected from the group consisting of dicalcium phosphate anhydrous, mannitol, hydroxypropyl cellulose, sodium stearyl fumarate, microcrystalline cellulose, starch, crospovidone, cross-linked sodium carboxymethyl cellulose and magnesium stearate.

5. The method for preparing an oral composite tablet according to claim 1, wherein the excipient of the step (2) includes one or more selected from the group consisting of dicalcium phosphate anhydrous, mannitol, hydroxypropyl cellulose, sodium stearyl fumarate, colloidal silicon dioxide, microcrystalline cellulose, pregelatinized starch, crospovidone, cross-linked sodium carboxymethyl cellulose and magnesium stearate.

6. The method for preparing an oral composite tablet according to claim 1, wherein the dry granulating in the step (1) or (2) is performed using a roller compactor.

7. The method for preparing an oral composite tablet according to claim 1, wherein the pre-pressure of the step (3) is 0.1 to 1 kN.

8. The method for preparing an oral composite tablet according to claim 1, wherein the main pressure of the step (4) is 28 to 35 kN.

9. An oral composite tablet prepared according to any one of claims 1 to 8, which comprises:
an upper layer containing a proton pump inhibitor or a pharmaceutically acceptable salt thereof as an active ingredient; and
a lower layer containing an antacid or a pharmaceutically acceptable salt thereof as an active ingredient.

10. The oral composite tablet according to claim 9, wherein the proton pump inhibitor includes one or more selected from the group consisting of esomeprazole, omeprazole, lansoprazole, rabeprazole and pantoprazole.

11. The oral composite tablet according to claim 9, wherein the antacid includes one or more selected from the group consisting of magnesium hydroxide, magnesium oxide, sodium bicarbonate and potassium carbonate.

12. The oral composite tablet according to claim 9, wherein the oral composite tablet has a hardness of 12.8 to 15.5 kp.

13. The oral composite tablet according to claim 9, wherein the oral composite tablet has proton pump inhibitor dissolution rate of 10% to 50% for 10 minutes and antacid dissolution rate of 70% or higher at the same time when it is tested in a mixture of 0.1N HCl 75 mℓ and purified water 225 mℓ under a condition of a rotation speed of 7512 rpm and 37±0.5°C according to the paddle method, the second method of the United States Pharmacopoeia (USP) dissolution test.
